# EUROPÄISCHE PATENTSCHRIFT

(11) **EP 0 893 069 B1**
(45) Veröffentlichungstag und Bekanntmachung des Hinweises auf die Patenterteilung: **22.10.2003**
(21) Anmeldenummer: 98111614.8
(22) Anmeldetag: 24.06.1998
(51) Int. Cl.: A23L 1/305, A61K 38/17

(54) **Antioxidative Zusammensetzungen mit Süssmolkekonzentrat und Casein**
Antioxidant composition containing a concentrate of sweet whey and casein
Composition antioxydante comprenant un concentré de petit lait doux et de la caséine

(30) Priorität: 24.06.1997 DE 19726868
(43) Veröffentlichungstag der Anmeldung: 27.01.1999
(73) Patentinhaber: Jobst Krauskopf Marketing für Arznei- und Gesundheitsmittel, 29576 Barum Krs. Uelzen (DE)
(72) Erfinder: Krauskopf, Jobst, 29576 Barum/Krs. Uelzen (DE); Elstner, Erich F., Prof.Dr., 82194 Gröbenzell (DE)
(74) Vertreter: Hansen, Bernd, Dr. Dipl.-Chem.

(56) Entgegenhaltungen:
- EP-A- 0 046 326
- EP-A- 0 631 731
- EP-A- 0 709 034
- EP-A- 0 845 266
- WO-A-92/00994
- ALLEN J C ET AL: "Influence of milk proteins on lipid oxidation in aqueous emulsion I. casein, whey protein and alpha-lactalbumin" JOURNAL OF DAIRY RESEARCH, Bd. 49, 1982, Seiten 239-248, XP002097667
- REYNOLDS E C ET AL: "Effect of protein solutions on caries experience of the rat" J. DENT RES., Bd. 62, Nr. 4, 1983, Seite 409 XP002097668
- ROHNERT, UTE ET AL: "Health promoting effect of whey and casein preparations on free radical-induced pathogenic processes." INTERNATIONAL DAIRY JOURNAL, (MAY-JUNE, 1998) VOL. 8, NO. 5-6, PP. 574. MEETING INFO.: MEETING ON FUNCTIONAL FOODS: DESIGNER FOODS FOR THE FUTURE CORK, IRELAND SEPTEMBER 30-OCTOBER 2, 1997 , XP002097669

## Beschreibung

Die gesundheitsfördernde Wirkung von Molke ist seit dem Altertum bekannt. Schon vor mehr als zweitausend Jahren wurde von Molkekuren berichtet, in Europa waren sie vor allem in der Zeit vom 17. bis 19. Jahrhundert beliebt, wobei die Förderung des allgemein Wohlbefindens durch Entgiftung und Entschlackung im Vordergrund stand. Auch im kosmetischen Bereich fand Molke Verwendung, wobei neben Molkekuren auch Molkebäder verabreicht wurden. In jüngster Zeit wurde die Molke, vor allem zur Anwendung in der modernen Diätetik, wiederentdeckt.

Molke fällt bei der Käseherstellung aus Milch, wie z.B. Kuhmilch, Ziegenmilch, Schafsmilch, Büffelmilch und Kamelmilch, an, nachdem das in der Milch vorhandene Casein ausgefällt wurde. Nach Art der Gewinnung unterscheidet man Süßmolke, die als Milchserum nach enzymatischer Ausfällung von Casein durch Labferment entsteht, und Sauermolke, die nach Abtrennung des Caseins durch Säurefällung gewonnen wird. Die pH-Wert-Grenze zwischen Süß- und Sauermolke ist nicht ganz scharf umrissen und liegt allgemein über bzw. unter einem Bereich von 5,6 bis 5,9.

Molke enthält alle wasserlöslichen Komponenten der Milch, insofern diese nicht durch Lab oder Säure ausgefällt werden. Süßmolke enthält ca. 4,9% Lactose, 0,8% Protein, 0,5% Mineralien, 0,2% Milchfett und 0,2% Milchsäure. Wichtige Proteine der Molke sind α-Lactalbumin, β-Lactoglobulin, Lactoferrin, Blutserumalbumin, Immunglobuline und Lactoperoxidase. An Fetten liegen sowohl gesättigte als auch ein- und mehrfach ungesättigte Fettsäuren sowie Cholesterin vor.

Weitere wichtige Inhaltsstoffe der Molke sind Vitamine (z.B. Ascorbinsäure, Thiamin, Niacin, Pantothensäure, Retinol, Riboflavin, Pyridoxin und Cobalamin) und freie Aminosäuren.

Süßmolke kann weiterhin teilweise oder vollständig hydrolysiert vorliegen, d.h. die Zucker sind dann in die entsprechenden Einfachzucker gespalten. Derartige Hydrolysate sind von der erfindungsgemäßen Bedeutung der Begriffe Süßmolke und Süßmolkekonzentrat mit umfaßt.

Bei allen Mengenangaben kann der Gehalt der Molke je nach verwendeter Milch (z.B. Kuh, Ziege, Schaf) und nach hergestellter Käseart schwanken.

Aufgrund ihres Nährstoffgehalts eignet sich Molke zur Herstellung ernährungsphysiologisch wertvoller Produkte. Der hohe Lactosegehalt der Molke wirkt sich günstig auf die endogene Verwertung von Calcium, Natrium und Kalium aus der Nahrung aus, und die durch Molke erhöhte Vitamin- und Mineralstoffzufuhr ist insbesondere bei älteren mangelernährten Menschen wünschenswert.
Entzündungen entstehen durch schädliche Einwirkung auf Gewebe, wie z.B. Infektionen mit Mikroorganismen. Als Symptome treten u.a. Rötung, Schwellung, Überwärmung und Schmerz auf. Verantwortlich für diese Symptome sind Mediatorstoffe von unterschiedlichem chemischem Charakter, neben biogenen Aminen (z.B. Histamin), Peptiden (z.B. Bradykinin) und Produkten des Arachidonsäurestoffwechsels (z.B. Prostaglandine, Leukotriene) gehören dazu auch reaktive Sauerstoffspezies (Superoxidradikalanionen, Wasserstoffperoxid, Hydroxylradikale, Singulettsauerstoff).

Die Bildung dieser reaktiven Sauerstoffspezies geht von polymorphkernigen Leukozyten aus, die durch Kontakt mit einem phagozytierten Mikroorganismus in einen aktivierten Zustand übergehen. In diesem stimulierten Zustand überträgt eine NADPH-Oxidase in der Plasmamembran des Leukozyten Elektronen auf Sauerstoff, wobei zunächst das Superoxidradikalanion (O₂^{·-}) gebildet wird. Durch Folgereaktionen entstehen daraus Wasserstoffperoxid (H₂O₂) und Hydroxylradikale (OH^{·}). Diese reaktiven Sauerstoffspezies werden ins Phagosom abgegeben und dienen zur Abtötung des phagozytierten Mikroorganismus. Da die Freisetzung dieser Moleküle jedoch nicht nur ins Phagosom hinein geschieht, sondern auch nach "außen", kann es zur Schädigung des umliegenden Gewebes (u.a. Lipidperoxidation, Denaturierung von Enzymen, DNA-Strangbrüche) und somit zur Entstehung von Entzündungssymptomen und Gewebedegenerationen bis hin zu Nekrosen kommen.

Eine wesentliche, an den oxidativen Entzündungsprozessen beteiligte Grundreaktion ist die Generierung von Superoxidradikalalanionen bzw. der Folgeprodukte Wasserstoffperoxid und Hydroxylradikale durch NADPH-Oxidasen oder Xanthinoxidase. Die Xanthinoxidase-Reaktion ist das bedeutendste enzymatische System, in dem Fenton-Typ-Oxidationsmittel, insbesondere Fe⁺² und H₂O₂,gebildet werden. Aus diesen Spezies werden durch verschiedene Mechanismen OH-Radikale gebildet.

Die Erfinder haben festgestellt, daß die Bildung reaktiver Sauerstoffspezies von einer Zusammensetzung, die Süßmolkekonzentrat und Dipeptid enthält, inhibiert wird (EP-0 845 266).

Folglich besteht die Aufgabe der vorliegenden Erfindung darin, Süßmolkekonzentrat (SMK) enthaltende Zusammensetzungen mit antioxidativer Wirkung auf die epithelialen Kontakt- und Abwehrflächen aufzufinden, die diesen Effekt verbessern, insbesondere bei oraler Aufnahme oder topischer Anwendung.

Es wurde überraschend gefunden, daß die Zusammensetzung gemäß Anspruch 1, die SMK und Casein umfaßt, die Bildung von Fenton-Typ-Oxidantien durch das Xanthinoxidase-Reaktionssystem in wesentlich stärkerem Maße hemmt, als dies aus der Summe der Wirkungen von SMK bzw. Casein allein zu erwarten wäre. Die erfindungsgemäße Zusammensetzung enthält SMK und Casein in einem Gewichtsverhältnis von 1:2 bis 2:1, bevorzugt 1:1.

In einer weiteren Ausführungsform umfaßt die erfindungsgemäße Zusammensetzung weitere, zur Herstellung von diätetischen Lebensmitteln geeignete Zusatzstoffe. In einer weiteren Ausführungsform umfaßt die in Anspruch 1 beschriebene Zusammensetzung zusätzlich pharmazeutisch annehmbare Zusatzstoffe und/oder Trägermaterialien. Die erfindungsgemäße Zusammensetzung kann zur Herstellung von Lebensmitteln, wie z.B. diätetischen und/oder angereicherten Lebensmitteln, und Nahrungsergänzungsmitteln verwendet werden. Bevorzugte Beispiele für unter Verwendung der erfindungsgemäßen Zusammensetzung hergestellte Lebensmittel sind Milchprodukte, milchhaltige Lebensmittel bzw. Milchmischprodukte.

Weiterhin kann die erfindungsgemäße Zusammensetzung verwendet werden zur Herstellung einer pharmazeutischen Zusammensetzung zur Behandlung von Erkrankungen, die mit oxidativen Prozessen verbunden sind, bevorzugt zur Behandlung von Entzündungserkrankungen von Säugern, insbesondere des Menschen. Noch weiter bevorzugt ist die Verwendung zur Behandlung von Entzündungserkrankungen der epithelialen Kontakt- und Abwehrflächen, insbesondere des Mund-Rachenbereiches, des Nasen-Ohrenbereiches, der Atemwege und der Verdauungswege, sowie des Genitalbereich, insbesondere in der Frauenheilkunde.

Die erfindungsgemäße Zusammensetzung kann schließlich in verschiedenen Formen, z.B. in fester Form als Lutschtablette, Pulver oder Granulat, oder in flüssiger Form als Sirup oder Saft vorliegen.

Die Dosierung der erfindungsgemäßen Zusammensetzung kann je nach Alter, Gewicht und Geschlecht der zu behandelnden Person variieren. Bei der Verabreichung der erfindungsgemäßen Zusammensetzung an Säuger im allgemeinen erfolgt die Dosierung ebenfalls in Abhängigkeit von Tierart und Gewicht.

Die erfindungsgemäße Zusammensetzung kann in vorteilhafter Weise ebenfalls zur Herstellung von Haut- und Haarreinigungsmitteln wie Badezusätzen, Shampoos und Seifen verwendet werden. Weitere vorteilhafte Anwendungen bestehen in der Verwendung zur Herstellung von Dental- und Mundgesundheitsprodukten wie Zahnpasta und Mundwasser bzw. -spülungen. Schließlich kann die erfindungsgemäße Zusammensetzung vorteilhaft zur Herstellung von Haut- und Körperpflegeprodukten wie Cremes, Salben und Lotionen eingesetzt werden. In diesen Anwendungen wird die erfindungsgemäße Zusammensetzung bevorzugt in einem Anteil von 1 bis 10 Gew.-%, bezogen auf das resultierende Produkt, eingesetzt. Zur Herstellung solcher Produkte kann die erfindungsgemäße Zusammensetzung dabei mit handelsüblichen Produktgrundlagen, z.B. Shampoo-, Seifen- und Salbengrundlagen, verwendet werden.

### Beispiel

Die Inhibitionswirkung wurde in einem In-vitro-Teststystem geprüft, das die oxidativen Prozesse während einer Entzündung simulieren soll.

Zum Nachweis der gesundheitsfördernden Wirkung von SMK und/oder Casein bei Entzündungszuständen wurde das Methioninderivat Ketomethylthiobutyrat (KMB) als Indikator verwendet. Von diesem Biomolekül ist bekannt, daß es eine entscheidende Rolle in den dabei beteiligten Prozessen spielt.

**Tabelle 1:**

| Einfluß von Süßmolkekonzentrat (SMK) und/oder Casein auf die Hemmung der KMB-Fragmentierung durch die XOD-Reaktion: | | | | |
|---|---|---|---|---|
| | Kontrolle | Casein | SMK | Casein/SMK |
| Menge (mg) | | 0.2 | 0.2 | 0.2/0.2 |
| % Reaktion | 100 | 93 | 90 | 35 |

Die Werte für die Reaktion (in % Reaktion relativ zur Kontrollprobe) sind Mittelwerte aus jeweils vier Einzelmessungen. Die Standardabweichung lag für alle Werte unter 10%.

Es wurde folgender Ansatz verwendet: 2 ml einer Reaktionslösung mit 0,1 M Phosphatpuffer mit einem pH-Wert von 7,4, 0,5 mM Xanthin, 0,33 U XOD (Boehringer), und 1,5 mM KMB. Die Reaktion wurde für 60 Minuten durchgeführt, und dann die im Gasraum des Reaktionsgefäßes befindliche Menge an gebildetem Ethen wie oben beschrieben quantifiziert. Die Ethen-Freisetzungsrate eines Ansatzes ohne Zugabe potentieller Inhibitorstoffe entspricht einer Grundrate von 100% (0% Hemmung).
Aus der Tabelle ist ersichtlich, daß der Inhibitionseffekt der Mischung aus SMK und Casein deutlich höher ist als die Summe der Inhibitionseffekte der einzelnen Bestandteile. Es kann also nicht von einem rein additiven Effekt der getesteten Einzelsubstanzen gesprochen werden, sondern vielmehr zeigt die Mischung einen überraschenden synergistischen Effekt.

Das oben beschriebene In-vitro-Testsystem zeigt, daß die antioxidative Wirkung von Süßmolke in einer Zusammensetzung, die neben Süßmolke ferner Casein umfaßt, in deutlich überadditiver Weise zunimmt.

## Patentansprüche

1. Zusammensetzung mit antioxidativer Wirkung auf die epithelialen Kontakt- und Abwehrflächen, umfassend Süßmolkekonzentrat und Casein in einem Verhältnis von 1:2 bis 2:1.

2. Zusammensetzung gemäß Anspruch 1, wobei das Süßmolkekonzentrat ein Konzentrat aus teilweise oder vollständig hydrolysierter Süßmolke ist.

3. Zusammensetzung gemäß Anspruch 1 oder 2, wobei das Süßmolkekonzentrat aus Kuhmilch, Ziegenmilch, Schafsmilch, Büffelmilch oder Kamelmilch gewonnen wird.

4. Zusammensetzung gemäß einem der Ansprüche 1-3, wobei die Zusammensetzung ferner Zusatzstoffe enthält, die zur Herstellung von Lebensmitteln, insbesondere diätetischen Lebensmitteln, und Nahrungsergänzungsmitteln geeignet sind.

5. Zusammensetzung gemäß einem der Ansprüche 1-4, wobei die Zusammensetzung ferner pharmazeutisch annehmbare Zusatzstoffe und/oder Trägermaterialien umfasst.

6. Zusammensetzung gemäß einem der Ansprüche 1-5, wobei die Zusammensetzung in fester Form als Lutschtablette, Pulver oder Granulat, oder in flüssiger Form als Sirup oder Saft vorliegt.

7. Verwendung der Zusammensetzung gemäß einem der Ansprüche 1-5 zur Herstellung eines diätetischen Lebensmittels.

8. Verwendung der Zusammensetzung gemäß einem der Ansprüche 1-5 zur Herstellung einer pharmazeutischen Zusammensetzung zur Behandlung von Erkrankungen, die mit oxidativen Prozessen verbunden sind, bei Säugern.

9. Verwendung der Zusammensetzung gemäß Anspruch 8, wobei die Zusammensetzung zur Behandlung von Erkrankungen beim Menschen verwendet wird.

10. Verwendung der Zusammensetzung gemäß Anspruch 8 oder 9, wobei die Erkrankungen Entzündungen sind.

11. Verwendung der Zusammensetzung gemäß Anspruch 8 oder 9, wobei die Erkrankungen Entzündungen der epithelialen Kontakt- und Abwehrflächen, insbesondere des Bund-Rachenbereiches, des Nasen-Ohrenbereiches, der Atemwege und der Verdauungswege sind.

12. Verwendung der Zusammensetzung gemäß Anspruch 9, wobei es sich um entzündliche Erkrankungen im Genitalbereich, insbesondere in der Frauenheilkunde handelt.

13. Verwendung der Zusammensetzung gemäß einem der Ansprüche 1-4 zur Herstellung von Milchprodukten, milchhaltigen Lebensmitteln und Milchmischprodukten.

14. Verwendung der Zusammensetzung gemäß einem der Ansprüche 1-3 zur Herstellung von Haut- und Haareinigungsmitteln, Dental- und Mundgesundheitsprodukten, und Haut- und Körperpflegeprodukten.

## Claims

1. Composition having anti-oxidative effect on the epithelial contact and defence surfaces, comprising sweet whey concentrate and casein in a ratio of 1: 2 to 2:1.

2. Composition according to claim 1, wherein the sweet whey concentrate is a concentrate of partly or completely hydrolysed sweet whey.

3. Composition according to claim 1 or 2, wherein the sweet whey concentrate is recovered from cow's milk, goat's milk, sheep's milk, buffalo's milk or camel's milk.

4. Composition according to one of claims 1-3, wherein the composition also contains additives, which are suitable for producing foodstuffs, in particular dietary foodstuffs, and dietary supplements.

5. Composition according to one of claims 1-4, wherein the composition also comprises pharmaceutically acceptable additives and/or excipients.

6. Composition according to one of claims 1-5, wherein the composition is present in solid form as a lozenge, powder or granules, or in liquid form as a syrup or juice.

7. Use of the composition according to one of claims 1-5 for producing a dietary foodstuff.

8. Use of the composition according to one of claims 1-5 for producing a pharmaceutical composition for treating diseases which are associated with oxidative processes in mammals.

9. Use of the composition according to claim 8, wherein the composition is used for treating diseases in the human.

10. Use of the composition according to claim 8 or 9, wherein the diseases are inflammations.

11. Use of the composition according to claim 8 or 9, wherein the diseases are inflammations of the epithelial contact and defence surfaces, in particular of the collar-pharyngeal region, of the nose-ear region, of the respiratory passages and of the digestive tracts.

12. Use of the composition according to claim 9, wherein it is a question of inflammatory diseases in the genital region, in particular in gynaecology.

13. Use of the composition according to one of claims 1-4 for producing milk products, milk-containing foodstuffs and mixed milk products.

14. Use of the composition according to one of claims 1-3 for producing skin and hair cleansing agents, dental and oral-hygiene products, and skin and body care products.

## Revendications

1. Composition ayant un effet antioxydant sur les surfaces épithéliales de contact et de défense, comprenant un concentré de petit lait doux et de la caséine dans un rapport de 1:2 à 2:1.

2. Composition selon la revendication 1, dans laquelle le concentré de petit lait doux est un concentré de petit lait doux partiellement ou totalement hydrolysé.

3. Composition selon la revendication 1 ou 2, dans laquelle le concentré de petit lait doux est obtenu à partir de lait de vache, de lait de chèvre, de lait de brebis, de lait de buffle ou de lait de chamelle.

4. Composition selon l'une des revendications 1-3, dans laquelle la composition contient d'autres additifs qui conviennent à la fabrication de produits alimentaires, en particulier de produits alimentaires diététiques, et de suppléments nutritionnels.

5. Composition selon l'une des revendications 1-4, dans laquelle la composition comprend de plus d'autres additifs et/ou supports pharmaceutiquement acceptables.

6. Composition selon l'une des revendications 1-5, dans laquelle la composition se présente sous forme solide en tant que pastilles à sucer, poudres ou granulés, ou sous forme liquide en tant que sirop ou jus.

7. Utilisation d'une composition selon l'une des revendications 1-5 pour la fabrication d'un produit alimentaire diététique.

8. Utilisation d'une composition selon l'une des revendications 1-5 pour la fabrication d'une composition pharmaceutique pour le traitement de maladies liées à des procédés oxydatifs chez les mammifères.

9. Utilisation d'une composition selon la revendication 8, dans laquelle la composition est utilisée pour le traitement de maladies chez l'homme.

10. Utilisation d'une composition selon la revendication 8 ou 9, dans laquelle les maladies sont des inflammations.

11. Utilisation d'une composition selon la revendication 8 ou 9, dans laquelle les maladies sont des inflammations des surfaces épithéliales de contact et de défense, en particulier de la zone du pharynx, de la région du nez et des oreilles, des voies respiratoires et des voies digestives.

12. Utilisation d'une composition selon la revendication 9, dans laquelle il s'agit de maladies inflammatoires dans la région génitale, en particulier en gynécologie.

13. Utilisation d'une composition selon l'une des revendications 1-4 pour la fabrication de produits lactés, de produits alimentaires à base de lait et de produits mixtes à base de lait.

14. Utilisation d'une composition selon l'une des revendications 1-3 pour la fabrication de produits produits nettoyants pour la peau et les cheveux, de produits de soins buccodentaires et de produits de l'hygiène dermatologique et corporelle.
